# EUROPÄISCHE PATENTSCHRIFT

(11) **EP 3 127 513 B1**
(45) Veröffentlichungstag und Bekanntmachung des Hinweises auf die Patenterteilung: **09.10.2019**
(21) Anmeldenummer: 15179646.3
(22) Anmeldetag: 04.08.2015
(51) Int. Cl.: A61F 5/01, A43B 7/20

(54) **SYSTEM ZUR ORTHOPÄDISCHEN BEHANDLUNG VON VERLETZUNGEN UND/ODER FEHLSTELLUNGEN DES UNTERSCHENKELS UND/ODER DES SPRUNGGELENKS**
SYSTEM FOR ORTHOPEDIC TREATMENT OF INJURIES AND/OR MISALIGNMENT OF THE LOWER LEG AND/OR THE ANKLE JOINT
SYSTEME DE TRAITEMENT ORTHOPEDIQUE DE LESIONS ET/OU DE DEFORMATIONS DE LA JAMBE ET/OU DE LA CHEVILLE

(43) Veröffentlichungstag der Anmeldung: 08.02.2017
(73) Patentinhaber: Darco (Europe) GmbH, 82399 Raisting (DE)
(72) Erfinder: Dietrich, Thomas, 82399 Raisting (DE); Zhang, Wu, Huntigton, WV West Virginia 25701 (US)
(74) Vertreter: Rupprecht, Kay

(56) Entgegenhaltungen:
- US-A- 4 938 777
- US-A- 5 226 875
- US-A- 5 951 504
- US-A1- 2004 236 259
- US-A1- 2006 137 226
- US-A1- 2010 036 304

## Beschreibung

Die vorliegende Erfindung betrifft ein System zur orthopädischen Behandlung von Verletzungen und/oder Fehlstellungen des Unterschenkels und/oder des Sprunggelenks.

Die postoperative Behandlung sowie die Behandlung von Verletzungen und/oder Fehlstellungen des Fußes erfolgt unter Zuhilfenahme von Orthesen. Dadurch soll die Ruhigstellung der Gliedmaße sowie die Förderung des Heilungsverlaufes und die Wiederherstellung der Beweglichkeit erreicht werden.

Beispielsweise offenbart die Druckschrift US 5,370,133 A in diesem Zusammenhang einen orthopädischen Stiefel zur Ruhigstellung des Fußes, der an der Vorderseite eine Öffnung aufweist. Eine Schaumschicht als Innenschuh umfasst den Unterschenkel, das Fußgelenk und den Fuß, um den Tragekomfort der Orthese zu erhöhen. Eine speziell geformte Sohle des Stiefels erleichtert das Abrollen während des Gehens und dämpft Stöße ab. Verschlüsse am Stiefel dienen dazu, eine gewünschte Kompression auf den Fuß ausüben und damit eine Stabilisierung des Fußes erreichen zu können. Die Druckschrift US 4,938,777 und die Druckschrift US 2004/0236259 A1 offenbaren eine Stabilisationseinlage.

Trotz Innenschuh und Sohlen mit Schaumkernen ergeben sich bei derartigen stiefelförmigen Orthesen grundlegende Nachteile. Unter anderem führt ihre Ausgestaltung zu Einschränkungen der Mobilität und Flexibilität des Patienten. Insbesondere ist eine möglichst unbeeinträchtigte Bewegungsfreiheit für die Patienten relevant, da die Orthese über längere Zeiträume bzw. den Tagesverlauf hinweg getragen werden muss, um eine adäquate Stabilisation des Fußes und den damit einhergehenden Heilungsverlauf sicherstellen zu können.

Um Patienten den Alltag zu erleichtern, muss eine Orthese in ihrer Gesamtheit für den Einsatz sowohl in Innenbereichen, z.B. zu Hause, als auch in Außenbereichen einfach zu handhaben sein. Neben der Stabilisation des Unterschenkels und/oder des Sprunggelenks treten somit weitere Anforderungen auf, die eine geeignete Orthese erfüllen können muss. Diese Anforderungen betreffen Eigenschaften wie die Größe der Orthese, das Gewicht, die Dämpfung bei Bodenkontakt und die Sauberkeit der Orthese, insbesondere an Oberflächen mit regelmäßigem Bodenkontakt. Ferner ist es für die Anwendung bei einer Vielzahl von Patienten vorteilhaft, wenn sich eine Orthese auf patientenspezifische Bedürfnisse sowie den jeweiligen Behandlungsfortschritt anpassen lässt.

Der vorliegenden Erfindung liegt die Aufgabe zugrunde, eine Orthese bereitzustellen, welche eine einfache Handhabung gewährleistet und für eine Vielzahl von Patienten adaptierbar ist, um somit eine Optimierung des Komforts, der Mobilität, der Hygiene und des Kostenaufwands von orthopädischen Behandlungen zu erzielen.

Diese Aufgabe wird durch ein System gemäß dem unabhängigen Anspruch 1 gelöst, sowie durch Vorrichtungen gemäß der unabhängigen Ansprüche 11 und 12. Weitere vorteilhafte Ausbildungen des erfindungsgemäßen Systems sind in den abhängigen Patentansprüchen angegeben.

Das erfindungsgemäße System weist einen Schuh mit einer Schuhsohle und einem Oberschuh auf. Des Weiteren ist eine Stabilisationseinlage in dem System vorgesehen. Die Stabilisationseinlage des Systems ist erfindungsgemäß mit einem Sohlenelement und wenigstens einem Stabilisierungsmittel ausgestaltet. Das Stabilisierungsmittel dient insbesondere zur Aufnahme von Kräften im Bereich des Unterschenkels und/oder des Sprunggelenks, wobei die Stabilisationseinlage an einem Fuß anliegend vorgesehen ist, um eine Stabilisation des Unterschenkels und/oder des Sprunggelenks zu erzielen. Insbesondere können Kräfte nach Verletzungen, bei Fehlstellungen oder postoperativ durch die anliegende Stabilisationseinlage aufgenommen werden, um den Heilungsprozess zu unterstützen und den Patienten frühzeitig mobilisieren zu können.

Der Oberschuh ist dazu ausgebildet, den Fuß teilweise zu umschließen. Durch die offene bzw. verschließbare Seite des Oberschuhs ist die Stabilisationseinlage in den Schuh einführbar, während die Stabilisationseinlage an dem Fuß anliegt. Hierzu muss die Stabilisationseinlage nicht von dem Fuß entfernt bzw. abgenommen werden. Folglich ist während einer andauernden Behandlung ein kontinuierlicher Schutz des Unterschenkels und/oder des Sprunggelenks durch die Stabilisationseinlage gegeben. Ein vielfaches Neuanlegen und Neupositionieren der Stabilisationseinlage an dem Unterschenkel und/oder dem Sprunggelenk ist nicht notwendig. Ebenso besteht eine komfortable Möglichkeit zum schnellen und einfachen Anlegen sowie Ablegen des Schuhs für den Aufenthalt in Außenbereichen. Hinsichtlich der zweckmäßigen Aufgabe eines Schuhs, ist folglich ein geeigneter Bodenkontakt u.a. durch die Absorption von Stößen beim Gehen sichergestellt.

Außerdem kommt die Stabilisationseinlage und speziell das Sohlenelement nicht in Kontakt mit der Bodenoberfläche. Wenn der Patient mobilisiert wird und sich in Außenbereichen aufhält, wird somit eine verbesserte Hygiene sichergestellt. Die Stabilisationseinlage wird nicht verschmutzt und der Schuh kann von dem Stabilisationselement gelöst und gegebenenfalls komfortabel gereinigt werden. Dabei wird die Stabilisation des Fußes weder beim Tragen des Schuhs während des Aufenthalts in Außenbereichen noch nach dem Ablegen des Schuhs beeinträchtigt.

In einer Ausführungsform der vorliegenden Erfindung weist das System eine Einlegesohle für den Schuh auf, wobei die Einlegesohle aus dem Schuh entnehmbar bzw. in den Schuh einlegbar ist. Wenn die Stabilisationseinlage mit dem Schuh verbunden ist, liegt das Sohlenelement der Stabilisationseinlage zwischen der Einlegesohle und der Schuhsohle des Schuhs. Es besteht somit der erfindungsgemäße Vorteil, dass dem Patienten eine patientenindividuelle, ergonomische Auftrittsfläche beim Tragen der Einlegesohle in Kombination mit der Stabilisationseinlage zur Verfügung steht. Dieser Vorteil liegt dabei sowohl vor, wenn allein die Einlegesohle in Kombination mit der Stabilisationseinlage getragen wird, als auch wenn zusätzlich der Schuh getragen wird. Auf diese Weise kann eine optimale Lastverteilung entlang des Fußgewölbes sichergestellt und einer fehlerhaften Belastung des Unterschenkels und/oder des Sprunggelenks vorgebeugt werden.

Des Weiteren ist die Einlegesohle aus einer oder mehreren Schichten aufgebaut. Die eine oder mehrere Schichten können über ihre Fläche hinweg variierende mechanische Eigenschaften aufweisen. Ebenso ist es möglich, dass eine oder mehrere Schichten Aussparungen aufweisen, um Entlastungszonen entlang des Fußgewölbes zu erzeugen. Entsprechend ist das erfindungsgemäße System anhand der Einlegesohle individuell konfigurierbar.

In einer weiteren erfindungsgemäßen Ausführungsform ist die Stabilisationseinlage per Kraftschluss und/oder Formschluss mit dem Schuh verbindbar. Der Patient ist in der Lage, die Stabilisationseinlage und den Schuh miteinander zu verbinden und diese Verbindung per Kraftschluss und/oder Formschluss sicherzustellen bzw. zu sichern. Der Patient kann demgemäß bei Bedarf die kraft- und/oder formschlüssige Verbindung herbeiführen oder auflösen und somit den Schuh zweckmäßig, z.B. zum Aufenthalt bzw. zum Gehen in Außenbereichen, einsetzen. Der erfindungsgemäße Schuh übernimmt somit vollumfänglich die Aufgaben eines alltagsüblichen Schuhs und ermöglicht dem Patienten die gewohnte Mobilität und Flexibilität.

Hierzu weist der Oberschuh gemäß einer bevorzugten Ausführungsform wenigstens ein Verschlussmittel auf. Das Verschlussmittel ist zur Herstellung einer lösbaren Verbindung zwischen dem Schuh und der Stabilisationseinlage ausgelegt. Es ist derart vorgesehen, dass die Enden des Oberschuhs, der den Fuß lediglich teilweise umschließt, durch das Verschlussmittel lösbar miteinander verbunden werden können. In diesem Fall kann der Schuh nicht mehr von der an dem Fuß befestigten Stabilisationseinlage, gegebenenfalls in Kombination mit der Einlegesohle, getrennt werden. Die Komponenten bleiben miteinander verbunden, bis der Kraftschluss und/oder Formschluss durch das Verschlussmittels wieder gelöst wird.

Das Verschlussmittel kann hierzu in Form einer Federverbindung, eines Klettverschlusses, eines Reißverschlusses, einer Nut/Nase-Verbindung, eines Druckknopfes, einer Schnalle, eines Schürsenkels oder eines vergleichbaren Verschlussmittels vorgesehen sein. Vorzugsweise ist das Verschlussmittel variabel einstellbar.

Ziel ist es, die Verbindung bzw. den Kontakt zwischen Stabilisationseinlage und Schuh durch einen Kraft- und/oder Formschluss sicherzustellen. Erfindungsgemäß ist somit ein einfacher Verschluss des Oberschuhs gewährleistet, um die Stabilisationseinlage in Verbindung mit dem Schuh zweckmäßig verwenden zu können.

Eine weitere Ausführungsform des erfindungsgemäßen Systems weist wenigstens ein Stabilisierungsmittel, vorzugsweise zwei Stabilisierungsmittel, auf, welches sich in Längsrichtung des Unterschenkels erstreckt. Dabei umfasst das Stabilisierungsmittel vorzugsweise eine longitudinale Grundform. Somit ist gewährleistet, dass sich das Stabilisierungsmittel beliebig weit entlang des Unterschenkels erstrecken und zweckmäßig Kräfte entlang des Unterschenkels und/oder des Sprunggelenks aufnehmen bzw. ableiten kann. Zwei Stabilisierungsmittel sind dabei vorzugsweise symmetrisch zur Mittelebene des Schuhs positioniert. Über die longitudinale Grundform hinausgehend können die Stabilisierungsmittel asymmetrische Geometrien aufweisen, um eine optimale Passform für die Anatomie an der Außenseite und der Innenseite des Fußes sicherzustellen. Demgemäß kann der Unterschenkel und/oder das Sprunggelenk durch das Stabilisierungsmittel optimal gestützt und stabilisiert werden, um diese während des Behandlungszeitraumes vor unerwünschten Krafteinwirkungen zu schützen.

Die vorliegende Erfindung weist in einer weiteren Ausführungsform wenigstens ein Stabilisierungsmittel auf, das den Unterschenkel und/oder das Sprunggelenk wenigstens teilweise umfasst. Demgemäß umfasst das Stabilisierungsmittel vorzugsweise den Unterschenkel und/oder das Sprunggelenk lediglich derart, dass Kräfte präventiv vom Fuß fern gehalten und von der Stabilisationseinlage übernommen bzw. aufgenommen werden. Durch ein teilweises Umfassen des Unterschenkels und/oder des Sprunggelenks ist ein verbesserter Komfort gegeben, da ein Kontakt zwischen Stabilisationseinlage und Hautoberfläche minimiert wird und lediglich die zur Kraftaufnahme wesentlichen Bereiche des Fußes von der Stabilisationseinlage abgedeckt werden. Es wird gleichfalls einer vollständigen Entlastung des Fußes und einer damit einhergehenden Degeneration der Stützmuskulatur vorgebeugt. Es ist dabei auch möglich, anhand einer gezielten Reduktion der Kraftübernahme durch das Stabilisationsmittel Reize zur Heilungsbeschleunigung und/oder zum Muskelaufbau zu setzen.

Weiterhin kann eine lediglich teilweise den Unterschenkel und/oder das Sprunggelenk umfassende Stabilisationseinlage für eine Vielzahl patientenspezifischer Anatomien des Fußes eingesetzt werden. Es ergibt sich demnach eine Reduktion des Materials zur Herstellung des Stabilisierungsmittels, eine Reduktion anzubietender Größenmodelle der Stabilisationseinlage und in der Folge eine Reduktion der Herstellungs- und Behandlungskosten.

In einer weiteren Ausführungsform des erfindungsgemäßen Gegenstands ist die Stabilisationseinlage mit dem Sohlenelement und dem wenigstens einen Stabilisierungsmittel einteilig oder mehrteilig ausgebildet. Mit einer einteiligen Ausführung der Stabilisationseinlage ist eine höhere Gesamtsteifigkeit der Stabilisationseinlage erzielbar. Eine mehrteilige Ausführung bietet gleichfalls den Vorteil, das Sohlenelement und das wenigstens eine Stabilisierungsmittel individuell auf den Patienten abzustimmen und die Ergonomie bzw. den Tragekomfort für den Patienten optimieren zu können. Insbesondere sind die Komponenten kosteneffizient auf den Patienten abzustimmen, wenn das wenigstens eine Stabilisierungsmittel und das Sohlenelement für die mehrteilige Ausführung der Stabilisationseinlage in Form eines Baukastensystems aus standardisierten Größen der Einzelkomponenten zusammensetzbar ist. An den Einzelkomponenten der Stabilisationseinlage können entsprechende Elemente zur hinreichenden, mechanischen Verbindung untereinander vorgesehen sein.

Gemäß einer weiteren Ausführungsform weist die Stabilisationseinlage wenigstens ein Material auf, dass zur Stabilisation des Unterschenkels und/oder des Sprunggelenks geeignet ist. Bevorzugterweise werden gemäß dem erfindungsgemäßen Gegenstand Polymere und/oder Komposite, wie z.B. Faserverbundwerkstoffe mit Polymermatrix, eingesetzt. Des Weiteren sind im Sinne der vorliegenden Erfindung auch Metalle oder Metallverbindungen für die Stabilisationseinlage einsetzbar. Es ist ferner möglich, verschiedene Materialien miteinander zu kombinieren, um gewünschte mechanische Eigenschaften in verschiedenen Bereichen der Stabilisationseinlage zu erzielen. Insbesondere unter Anwendung einer Kombination verschiedener Materialien ist die Stabilisationseinlage für die Kraftaufnahme entlang des Unterschenkels und/oder des Sprunggelenks gezielt konstruktiv auslegbar.

Eine weitere erfindungsgemäße Ausführungsform sieht vor, dass auf der Außenseite des wenigstens einen Stabilisierungsmittels wenigstens eine erste Komponente eines Befestigungsmittels, insbesondere eine Komponente eines Klettverschlusses, befestigbar ist. Dabei ist die Stabilisationseinlage mit Hilfe der ersten und einer zweiten Komponente des Befestigungsmittels an dem Unterschenkel und/oder dem Sprunggelenk fixierbar. Vorteilhafterweise kann die Stabilisationseinlage auf einfache Weise passgenau an den Unterschenkel und/oder das Sprunggelenk angepasst und in gewünschter Position fixiert werden. Somit ist die Stabilisationseinlage für eine Vielzahl von patientenspezifischen Anatomien adaptierbar während stets ein optimaler Kontakt zwischen Stabilisationseinlage und Fuß gewährleistet ist. Es wird weiterhin auch der Komfort für den Patienten erhöht, da die Stabilisationseinlage bei Bedarf auf einfache Weise am Fuß angebracht und von diesem entfernt werden kann, wobei einem Verrutschen am Fuß im fixierten Zustand vorgebeugt wird.

Die vorliegende Erfindung sieht gemäß einer Ausführungsform die erste Komponente des Befestigungsmittels auf dem wenigstens einen Stabilisierungsmittel vor. Die erste Komponente erstreckt sich auf dem Stabilisierungsmittel vorzugsweise in Längsrichtung des Unterschenkels. Hierdurch ist gewährleistet, dass das wenigstens eine, vorzugsweise longitudinale Stabilisierungsmittel über seine gesamte Länge entlang des Unterschenkels mit Hilfe der ersten und zweiten Komponente des Befestigungsmittels sicher fixierbar ist.

Neben einem System sieht die vorliegende Erfindung in einem nebengeordneten Patentanspruch weiterhin eine Stabilisierungseinlage zu Verwendung in einem erfindungsgemäßen System vor. Dabei ist die Stabilisierungseinlage im Querschnitt durch eine Frontalebene, insbesondere durch eine Frontalebene des Stabilisierungsmittels, vorzugsweise mit einem U-förmigen Querschnitt vorgesehen. Hieraus ergibt sich der Vorteil, dass die Stabilisationseinlage den Fuß, insbesondere den Unterschenkel und/oder das Sprunggelenk, umgreift und somit ein sicherer Halt des Fußes innerhalb der Stabilisationseinlage gewährleistet ist. Es wird einem Abrutschen oder Verrutschen der Stabilisationseinlage von/an dem Fuß vorgebeugt, unabhängig von der Verwendung des erfindungsgemäßen Systems mit oder ohne Schuh.

In einem weiteren nebengeordneten Patentanspruch wird ein Verbindungsmittel zur Verwendung in einem erfindungsgemäßen System beansprucht. Das Verbindungsmittel ist insbesondere dazu ausgebildet, die Einlegesohle mit der Schuhsohle und/oder die Stabilisationseinlage mit der Schuhsohle lösbar zu verbinden. Die wenigstens eine Verbindung ist dabei kraftschlüssig und/oder formschlüssig ausgelegt. Das erfindungsgemäße Verbindungsmittel bietet somit die Möglichkeit, die Komponenten des erfindungsgemäßen Systems direkt miteinander mechanisch zu verbinden. Diese direkte Verbindung ist dabei unabhängig von dem Verschlussmittel des Oberschuhs und kann in Form einer Federverbindung, eines Klettverschlusses, eines Reißverschlusses, einer Nut/Nase-Verbindung, eines Druckknopfes, einer Schnalle, eines Schürsenkels oder eines vergleichbaren Mittels erfolgen.

Die vorliegende Erfindung wird im Folgenden anhand von Ausführungsbeispielen unter Bezug auf die beigefügten schematischen Zeichnungen näher erläutert. Weitere Ausführungsformen des erfindungsgemäßen Gegenstandes sollen im Sinne der vorliegenden Erfindung hierdurch nicht ausgeschlossen sein.

Es zeigen schematisch:
- Fig. 1:: eine isometrische Frontansicht des erfindungsgemäßen Systems mit dem Schuh, der Stabilisationseinlage und der Einlegesohle;
- Fig. 2:: eine isometrische Explosionsdarstellung des erfindungsgemäßen Systems mit dem Schuh, der Stabilisationseinlage und einer individualisierten Einlegesohle;
- Fig. 3:: eine Seitenansicht des erfindungsgemäßen Systems mit dem Stabilisierungsmittel der Stabilisationseinlage und dem Schuh.

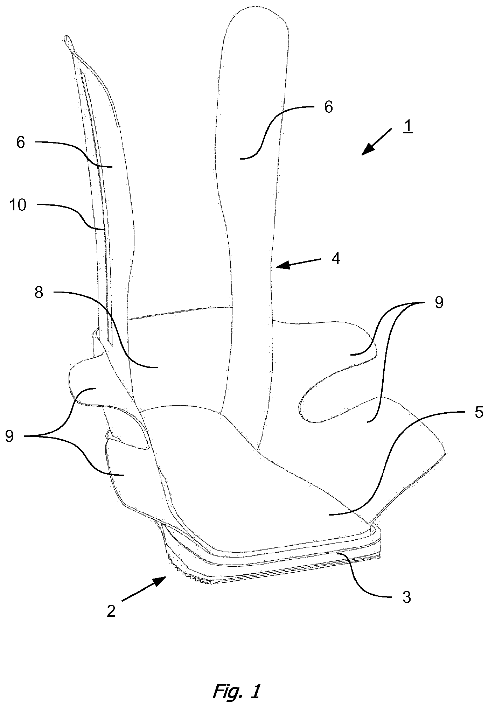

Fig. 1 zeigt das erfindungsgemäße System 1 mit dem Schuh 2 und der Stabilisationseinlage 4. Die U-förmige Stabilisationseinlage 4 ist gemäß der dargestellten Ausführungsform mit zwei Stabilisierungsmitteln 6 vorgesehen, die den Fuß wenigstens teilweise umschließen und sich longitudinal in Längsrichtung des Unterschenkels erstrecken. Die beiden Stabilisierungsmittel 6 weisen dabei eine Symmetrie zur Mittelebene des Schuhs 2 auf.

Weiterhin ist auf den Stabilisierungsmitteln 6 ein Befestigungsmittel 10 in Form einer ersten Komponente eines Klettverschlusses vorgesehen. Diese erste Komponente des Befestigungsmittels 10 erstreckt sich auf dem Stabilisierungsmittel 6 in Längsrichtung des Unterschenkels, gemäß der longitudinalen Grundform des Stabilisierungsmittels 6. Somit ist gewährleistet, dass das Stabilisierungsmittel 6 anhand der ersten und zweiten Komponente des Befestigungsmittels 10 über seine gesamte Länge an dem Unterschenkel sicher fixiert werden kann. Diese zweite, in Fig. 1 nicht dargestellte Komponente eines Klettverschlusses bzw. Befestigungsmittels 10 kann im Wesentlichen quer bzw. senkrecht zu der ersten Komponente an den Unterschenkel und/oder das Sprunggelenk angelegt werden, wobei zwischen erster und zweiter Komponente eine formschlüssige und/oder kraftschlüssige Verbindung entsteht.

Ferner weist der Schuh gemäß Fig. 1 eine Schuhsohle 3 auf, die in Verbindung zu der Stabilisierungseinlage 4 und der Einlegesohle 5 steht. Die gezeigte Einlegesohle 5 besitzt in der dargestellten Form keine individualisierte Form der Auftrittsfläche, sodass sie für eine Vielzahl von Patienten anwendbar ist.

Der Schuh 2 umfasst neben der Schuhsohle 3 im Weiteren den Oberschuh 8, der den Fuß teilweise umfasst. Der in Fig. 1 gezeigte Oberschuh 8 weist insbesondere eine Rückwand zur Abstützung der Ferse sowie Seitenwände auf. Außerdem weist der Oberschuh 8 Verschlussmittel 9 auf. Anhand der offenen Frontseite des Oberschuhs 8 ist der Patient in der Lage, den Schuh 3 mit der Stabilisationseinlage 4 und der Einlegesohle 5 zu verbinden, ohne dabei die Stabilisationseinlage 4 zusammen mit der Einlegesohle 5 vom Fuß lösen zu müssen. Insbesondere bei zweckmäßiger Verwendung ergibt sich beim Anlegen des Schuhs 2 eine Verbindung zwischen Schuh 2 bzw. Schuhsohle 3, der am Fuß vorgesehenen Stabilisationseinlage 4 und der Einlegesohle 5, wobei der Oberschuh 8 mit den Verschlussmitteln 9 an dem Fuß befestigt werden kann. Es liegt ein Kraftschluss und/oder Formschluss vor, der es bei zweckmäßiger Anwendung des Systems am Fuß unmöglich macht, die Komponenten voneinander zu trennen bis der Formschluss und/oder Kraftschluss durch das Öffnen der Verschlussmittel 9 wieder aufgelöst wird.

Es ergibt sich für die in Fig. 1 gezeigte Ausführungsform des erfindungsgemäßen Gegenstands eine komfortable und individualisierbare Lösung, um Patienten einen zeitweise einsetzbaren Schuh 2 zur Verfügung zu stellen, ohne dabei auf eine kontinuierliche Stabilisation des Unterschenkels und/oder des Sprunggelenks verzichten zu müssen.

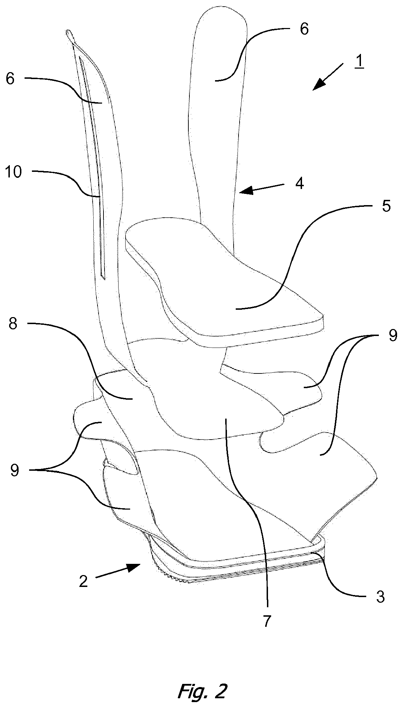

Ferner ist in Fig. 2 das erfindungsgemäße System 1 in einer Explosionsdarstellung gezeigt, wobei die Einlegesohle 5 eine individualisierbare Auftrittsfläche aufweist, sodass patientenspezifische Bedürfnisse des Fußgewölbes berücksichtigt und eine optimierte Ergonomie des erfindungsgemäßen Systems 1 zur Verfügung gestellt wird. In Abhängigkeit des spezifischen Krankheitsbildes eines Patienten können demnach patientenspezifische Anforderungen mit dem System 1 erfüllt werden, um den Behandlungs- und Heilungsverlauf zu unterstützen. Da das erfindungsgemäße System 1 aus einer oder mehreren Komponenten zusammengestellt wird, insbesondere einer einteiligen oder mehrteiligen Stabilisationseinlage 4 und/oder einer anpassbaren Einlegesohle 5 aus mehreren Schichten, können unter Anwendung eines Baukastenprinzips für standardisierte Komponentengrößen auch Behandlungs- bzw. Herstellungskosten gesenkt werden.

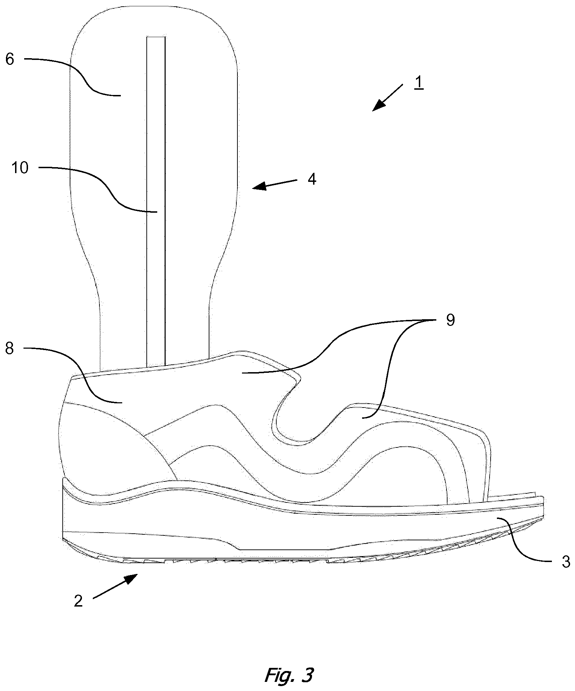

Die in Fig. 3 dargestellte Seitenansicht des erfindungsgemäßen Systems 1 zeigt den Oberschuh 3 mit dem an der Vorderseite vorgesehenen Verschlussmittel 9. Die Verschlussmittel 9 sind hierbei geschlossen, sodass eine form- und/oder kraftschlüssige Verbindung zwischen der Einlegesohle 5, sofern diese vorgesehen ist, der Stabilisationseinlage 4 und dem Schuh 2 bei zweckmäßiger Verwendung des Systems 1 am Fuß entsteht. Im Weiteren wird aus Fig. 3 ersichtlich, dass die Schuhsohle 3 speziell geformt sein kann, um den Patienten beim Gehen zu unterstützen und somit den Heilungs- bzw. Behandlungsverlauf zu optimieren. Gleichfalls ist auch vorstellbar, spezifisch ausgestalte Schuhsohlen 3 je nach Art und Fortschritt der Behandlung in dem System 1 einzusetzen.

### Bezugszeichenliste

- 1:: System
- 2:: Schuh
- 3:: Schuhsohle
- 4:: Stabilisationseinlage
- 5:: Einlegesohle
- 6:: Stabilisierungsmittel
- 7:: Sohlenelement
- 8:: Oberschuh
- 9:: Verschlussmittel
- 10:: Befestigungsmittel

## Patentansprüche

1. System (1) zur orthopädischen Behandlung von Verletzungen und/oder Fehlstellungen des Unterschenkels und/oder des Sprunggelenks, mit einem Schuh (2), welcher eine Schuhsohle (3) und einen Oberschuh (8) aufweist, und mit einer Stabilisationseinlage (4), wobei die Stabilisationseinlage (4) ein Sohlenelement (7) und wenigstens ein Stabilisierungsmittel (6) zur Aufnahme von Kräften im Bereich des Unterschenkels und/oder des Sprunggelenks aufweist, wobei die Stabilisationseinlage (4) an einem Fuß anliegend vorgesehen und der Oberschuh (8) des Schuhs (2) dazu ausgebildet ist, den Fuß teilweise zu umschließen, sodass die Stabilisationseinlage (4) mit dem Schuh (2) verbindbar ist, **dadurch gekennzeichnet, dass** der Oberschuh (8) eine verschließbare Seite aufweist, über die die Stabilisationseinlage (4) in den Schuh (2) einführbar ist, während die Stabilisationseinlage (4) an dem Fuß anliegt.

2. System (1) nach Anspruch 1,
**dadurch gekennzeichnet, dass**
das System (1) ferner eine Einlegesohle (5) für den Schuh (2) aufweist und das Sohlenelement (7) der Stabilisationseinlage (4) bei Verbindung der Stabilisationseinlage (4) mit dem Schuh (2) vorzugsweise zwischen der Einlegesohle (5) und der Schuhsohle (3) vorgesehen ist.

3. System (1) nach Anspruch 1 oder 2,
**dadurch gekennzeichnet, dass**
die Stabilisationseinlage (4) per Kraftschluss und/oder Formschluss mit dem Schuh (2) verbindbar ist.

4. System (1) nach einem der vorhergehenden Ansprüche,
**dadurch gekennzeichnet, dass**
der Oberschuh (8) wenigstens ein Verschlussmittel (9) aufweist, zur Herstellung einer lösbaren Verbindung zwischen dem Schuh (2) und der am Fuß anliegend vorgesehenen Stabilisationseinlage (4).

5. System (1) nach einem der vorhergehenden Ansprüche,
**dadurch gekennzeichnet, dass**
das wenigstens eine Stabilisierungsmittel (6) sich in Längsrichtung des Unterschenkels erstreckt und eine vorzugsweise longitudinale Grundform aufweist.

6. System (1) nach einem der vorhergehenden Ansprüche,
**dadurch gekennzeichnet, dass**
das wenigstens eine Stabilisierungsmittel (6) den Unterschenkel und/oder das Sprunggelenk wenigstens teilweise umfasst.

7. System (1) nach einem der vorhergehenden Ansprüche,
**dadurch gekennzeichnet, dass**
die Stabilisationseinlage (4) mit dem Sohlenelement (7) und dem wenigstens einen Stabilisierungsmittel (6) einteilig oder mehrteilig ausgebildet ist.

8. System (1) nach einem der vorhergehenden Ansprüche,
**dadurch gekennzeichnet, dass**
die Stabilisationseinlage (4) wenigstens ein Material aufweist, das zur Stabilisation des Unterschenkels und/oder des Sprunggelenks geeignet ist, vorzugsweise ein Polymer und/oder ein Komposit.

9. System (1) nach einem der vorhergehenden Ansprüche,
**dadurch gekennzeichnet, dass**
auf der Außenseite des wenigstens einen Stabilisierungsmittels (6) wenigstens eine erste Komponente eines Befestigungsmittels (10), insbesondere eine Komponente eines Klettverschlusses, befestigbar ist, sodass die Stabilisationseinlage (4) mit Hilfe der ersten und einer zweiten Komponente des Befestigungsmittels (10) an dem Unterschenkel und/oder Sprunggelenk fixierbar ist.

10. System (1) nach einem der vorhergehenden Ansprüche,
**dadurch gekennzeichnet, dass**
sich die erste Komponente des Befestigungsmittels (10) auf dem Stabilisierungsmittel (6) vorzugsweise in Längsrichtung des Unterschenkels erstreckt.

## Claims

1. A system (1) for the orthopedic treatment of injuries and/or misalignments of the lower leg and/or the ankle joint comprising a shoe (2) having a shoe sole (3), an upper shoe (8) and a stabilizing insert (4), wherein the stabilizing insert (4) comprises a sole element (7) and at least one stabilizing means (6) for absorbing forces in the area of the lower leg and/or ankle joint, wherein the stabilizing insert (4) is provided adjacent to a foot and the upper shoe (8) of the shoe (2) is designed to partially enclose the foot so that the stabilizing insert (4) can be connected to the shoe (2),
**characterized in that**
the upper shoe (8) has a closeable side via which the stabilizing insert (4) can be inserted into the shoe (2) while the stabilizing insert (4) rests against the foot.

2. The system (1) according to claim 1,
**characterized in that**
the system (1) further comprises an inlay sole (5) for the shoe (2) and the sole element (7) of the stabilizing insert (4) is preferably provided between the inlay sole (5) and the shoe sole (3) when the stabilizing insert (4) is connected to the shoe (2).

3. The system (1) according to claim 1 or 2,
**characterized in that**
the stabilizing insert (4) can be connected to the shoe (2) by non-positive connection and/or positive connection.

4. The system (1) according to one of the preceding claims,
**characterized in that**
the upper shoe (8) comprises at least one closure means (9) for establishing a releasable connection between the shoe (2) and the stabilizing insert (4) provided adjacent to the foot.

5. The system (1) according to one of the preceding claims,
**characterized in that**
the at least one stabilizing means (6) extends in the longitudinal direction of the lower leg and preferably exhibits a basic longitudinal form.

6. The system (1) according to one of the preceding claims,
**characterized in that**
the at least one stabilizing means (6) at least partly surrounds the lower leg and/or the ankle joint.

7. The system (1) according to one of the preceding claims,
**characterized in that**
the stabilizing insert (4) with the sole element (7) and the at least one stabilizing means (6) is of one-piece or multi-piece configuration.

8. The system (1) according to one of the preceding claims,
**characterized in that**
the stabilizing insert (4) comprises at least one material suitable for stabilizing the lower leg and/or the ankle joint, preferably a polymer and/or a composite.

9. The system (1) according to one of the preceding claims,
**characterized in that**
at least one first component of a fastening means (10), in particular a component of a Velcro fastener, is affixable to the external side of the at least one stabilizing means (6) so that the stabilizing insert (4) can be secured to the lower leg and/or ankle joint by means of the first and a second component of the fastening means (10).

10. The system (1) according to one of the preceding claims,
**characterized in that**
the first component of the fastening means (10) on the stabilizing means (6) preferably extends in the longitudinal direction of the lower leg.

## Revendications

1. Système (1) pour le traitement orthopédique de blessures et/ou de malpositions de la jambe et/ou de l'articulation de la cheville, comprenant une chaussure (2) qui présente une semelle de chaussure (3) et une partie supérieure de chaussure (8) et un insert de stabilisation (4),
l'insert de stabilisation (4) présentant un élément de semelle (7) et au moins un moyen de stabilisation (6) pour absorber des forces dans la zone de la jambe et/ou de l'articulation de la cheville, l'insert de stabilisation (4) étant prévu en contact avec un pied et la partie supérieure (8) de la chaussure (2) est réalisée pour entourer partiellement le pied afin que l'insert de stabilisation (4) puisse être relié à la chaussure (2), **caractérisé en ce que** la partie supérieure de chaussure (8) présente un côté pouvant être fermé ,par lequel l'insert de stabilisation (4) peut être introduit dans la chaussure (2) lorsque que l'insert de stabilisation (4) est en contact avec le pied.

2. Système (1) selon la revendication 1, **caractérisé en ce que** le système (1) comprend en outre une semelle intérieure (5) pour la chaussure (2) et l'élément de semelle (7) de l'insert de stabilisation (4) est prévu de préférence entre la semelle intérieure (5) et la semelle de chaussure (3) lorsque la semelle intérieure de stabilisation (4) est reliée à la chaussure (2).

3. Système (1) selon la revendication 1 ou 2, **caractérisé en ce que** l'insert de stabilisation (4) peut être relié à la chaussure (2) par coopération de forces et/ou par coopération de formes.

4. Système (1) selon l'une des revendications précédentes, **caractérisé en ce que** la partie supérieure de chaussure (8) présente au moins un moyen de fermeture (9), pour réaliser une liaison amovible entre la chaussure (2) et l'insert de stabilisation (4) prévu en contact avec le pied.

5. Système (1) selon l'une des revendications précédentes, **caractérisé en ce que** ledit au moins un moyen de stabilisation (6) s'étend dans la direction longitudinale de la jambe et présente une forme de base de préférence longitudinale.

6. Système (1) selon l'une des revendications précédentes, **caractérisé en ce que** ledit au moins un moyen de stabilisation (6) entoure au moins partiellement la jambe et/ou l'articulation de cheville.

7. Système (1) selon l'une des revendications précédentes, **caractérisé en ce que** l'insert de stabilisation (4) est réalisé d'un seul tenant ou en plusieurs parties avec ledit au moins un élément de semelle (7) et ledit au moins un moyen de stabilisation (6).

8. Système (1) selon l'une des revendications précédentes, **caractérisé en ce que** l'insert de stabilisation (4) comprend au moins un matériau, de préférence un polymère et/ou un composite, qui est approprié pour stabiliser la jambe et/ou l'articulation de la cheville.

9. Système (1) selon l'une des revendications précédentes, **caractérisé en ce que** sur la face extérieure dudit au moins un moyen de stabilisation (6) peut être fixé au moins un premier composant d'un moyen de fixation (10), en particulier un composant d'une fermeture auto-agrippante, de telle sorte que l'insert de stabilisation (4) peut être fixé sur la jambe et/ou sur l'articulation de la cheville à l'aide du premier et d'un second composant du moyen de fixation (10).

10. Système (1) conformément à l'une des revendications précédentes, **caractérisé en ce que** le premier composant du moyen de fixation (10) s'étend sur le moyen de stabilisation (6), de préférence dans le sens longitudinal de la jambe.
